# EUROPEAN PATENT SPECIFICATION

(11) **EP 1 539 249 B1**
(45) Date of publication and mention of the grant of the patent: **09.07.2008**
(21) Application number: 03765080.1
(22) Date of filing: 22.07.2003
(51) Int. Cl.: A61K 49/12, A61K 49/18

(54) **PROCEDURES OF CELLULAR LABELLING WITH PARAMAGNETIC COMPLEXES FOR MRI APPLICATIONS**
ZELLMARKIERUNGSVERFAHREN MIT PARAMAGNETISCHEN KOMPLEXEN FÜR MRT-ANWENDUNGEN
PROCEDURE DE MARQUAGE CELLULAIRE AVEC DES COMPLEXES PARAMAGNETIQUES EN VUE D'APPLICATIONS IRM

(30) Priority: 22.07.2002 US 397000 P
(43) Date of publication of application: 15.06.2005
(73) Proprietor: BRACCO IMAGING S.p.A., 20134 Milano (IT)
(72) Inventor: AIME, Silvio, I-20134 Milano (IT); GENINATTI CRICH, Simonetta, I-10143 TORINO (IT); LATTUADA, Luciano, I-20134 Milano (IT)
(74) Representative: Minoja, Fabrizio
(86) International application number: PCT/EP2003/007962
(87) International publication number: WO 2004/009134

(56) References cited:
- WO-A-01/52906
- WO-A-87/02893
- WO-A-92/21017
- WO-A-99/21592
- WO-A-99/30745
- TOKUMITSU H ET AL: "DESIGN AND PREPARATION OF GADOLINIUM-LOADED CHITOSAN PARTICLES FOR CANCER NEUTRON CAPTURE THERAPY" STP PHARMA SCIENCES, PARIS, FR, vol. 10, no. 1, 2000, pages 39-49, XP000951450 ISSN: 1157-1489
- KABALKA G W ET AL: "GADOLINIUM-LABELED LIPOSOMES CONTAINING PARAMAGNETIC AMPHIPATHIC AGENTS: TARGETED MRI CONTRAST AGENTS FOR THE LIVER" MAGNETIC RESONANCE IN MEDICINE, ACADEMIC PRESS, DULUTH, MN, US, vol. 8, no. 1, 1 September 1988 (1988-09-01), pages 89-95, XP002054627 ISSN: 0740-3194
- LOUIE ANGELIQUE Y ET AL: "In vivo visualization of gene expression using magnetic resonance imaging" NATURE BIOTECHNOLOGY, NATURE PUBLISHING, US, vol. 18, March 2000 (2000-03), pages 321-325, XP002181437 ISSN: 1087-0156

## Description

### SUMMARY OF THE INVENTION

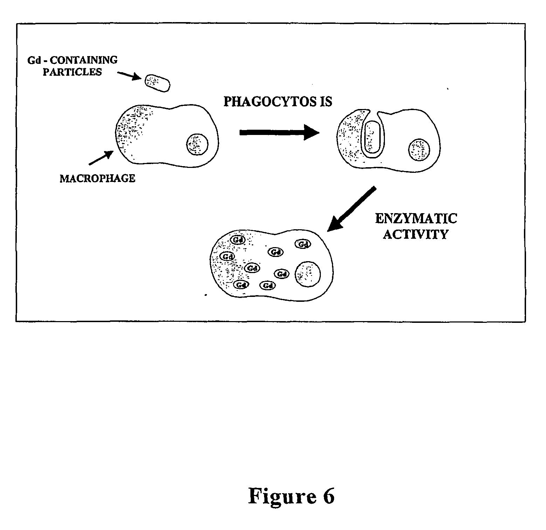

MRI visualisation of cellular systems requires internalisation of a large number of paramagnetic complex units. It has been found that the most efficient route for introducing large amounts of metal complexes into a cell is to internalise them in the form of finely divided particulate. Therefore, internalisation may be carried out, depending on the characteristics of the cell type, through: a) phagocytic activity or b) receptor-stimulated endocytosis. In the latter case, units responsible for targeting the cell surface will have to be bound to the particulate surface, which will optionally be coated by means of procedures similar to those used for the iron oxides commonly employed as MRI contrast agents. Once internalised, the particles will be gradually degraded by the activity of appropriate enzymes or anyhow by suitable effectors inside the surrounding micro-environment, thus releasing the single unities of the paramagnetic complex as schematically disclosed in Fig. 6. The presence of the particulate in the cell can easily be detected with the MRI technique by T2-weighted sequences, while the presence of free complex units, also called MR-Imaging Probes, is more easily revealed by T1-weighted sequences. The enzyme gradually degrading the particles may naturally occur in the concerned cell or it may be specifically expressed by known molecular biology procedures or it may be administered in a form which localises in the concerned cellular compartments.

### BACKGROUND OF THE INVENTION

Paramagnetic complexes of transition metals (particularly Mn(II) and Mn(III)) and of lanthanides (particularly Gd(III)) are used as Contrast Agents (CA) in Magnetic Resonance Imaging due to their ability of inducing a remarkable enhancement in the relaxation rate of tissue protons. In most cases they have extracellular distribution and have proved particularly valuable for evaluating organs and tissues perfusion, or for revealing any abnormalities in the hemato-encephalic barrier and so on. In angiographic applications, CA segregation in the vasal compartment through non-covalent interaction with serum albumin is sought. In less cases, the diagnostic potential is related to the intracellular distribution of the CA, as is the case with hepato-specific agents which, upon active transport, enter the hepatocytes but not metastases which may be present in this organ. A further example is the intemalisation of a paramagnetic complex inside red blood cells by osmotic shock. This procedure provides red blood cells containing a large number of paramagnetic complexes, which may give important information on flow, volume, etc. in MRI imaging.

Recently, attention has been focused on the possibility of labelling specific cell types in order to visualise them in MRI procedures. An example of application connected with an improvement in current diagnostic protocols relates to the targeting on the receptors that are over-expressed in tumour cells. In these cases, cellular labelling can be carried out either through accumulation of CA on the outer surface of the cell membrane, or through internalisation by means of suitable regenerable receptors. Other applications of great interest concern the possibility of monitoring cellular homing, with interesting perspectives in tracking stem cells. Highly innovative is also the possibility of using cellular labelling to evidence occurred transfection in gene therapy. Targeting of therapeutic genes is an extremely interesting possibility and related studies are expected to markedly develop in the future. Independently of the gene therapy technology which will prevail (virus or proteins/DNA complexes or liposomes/DNA adducts) it will be necessary to develop suitable gene transfer indicators. To date, "in vitro" studies rely on procedures involving the use of genes that express enzymes, such as β-galactasidase or a green fluorescent protein, in the infected cells. In other words, in the infection procedure of a given cell, use is made of a construct containing not only the gene specific for the treatment of the diseases, but also the gene for the indicator that will provide information on the occurred transfer of the administered gene material into the nucleus. Due to the high spacial resolution ability of MRI, this procedure has of course been considered to monitor gene transfer processes. Meade et coll. used a Gd-HPDO3A derivative containing one galactose unit cleaved by the enzyme β-galactosidase. The transformation induces a change of the complex relaxivity which can be revealed by MR image. In practice, this approach is apparently limited by several factors, such as the limited relaxivity enhancement and, even more, by the difficulties in specifically introducing the complex in the concerned cells (in the paper by Meade et coll. the complex is directly injected into the oocytes transfected with the β-galactosidase gene). Other Authors suggested the use of magnetite particles as gene transfer indicators by means of overexpressed transferrin receptors. The concerned protein acts as a carrier for the internalisation of the magnetic particle. One limit to this approach is that it is preferable to observe a positive contrast enhancement rather than a negative one, as is the case with these particles acting on the change in magnetic susceptivity. Moreover, the cell-internalization of large amount of iron yields serious concern about its metabolic fate.

Methods of detecting enzyme activity or binding to the target molecules by MRI techniques are disclosed in Nature Biotech, Nature Publishing, US, vol. 18, March 2000 (2000-03), pp. 321-325 and in WO 99/21592. According to such methods, MRI contrast agents comprising a blocking group are used. The blocking group is cleaved by the enzyme to be monitored, whereby the deblocked contrast agent enters into direct interaction with water protons and, thus, shows increased relaxivity. In the method of WO 01/52906, a contrast agent comprising a masking polypeptide is used; after enzymatic cleavage of the polypeptide, the contrast agent binds to the target molecule, thereby producing a rise in relaxivity.

Therefore, there is a need for developing new cellular labelling strategies based on the release of paramagnetic complexes (positive contrast) endowed with specific tropism towards the concerned cells and able to attain a high contrast in the resulting MRI images,

### DESCRIPTION OF THE INVENTION

As mentioned in the introduction, a gene encoding for an indicator enzyme is often used in cellular labelling procedures. On the other hand detection of a given enzyme overexpression is often of high diagnostic value in a number of pathologies. For MRI observations it is necessary that the presence of a given enzyme induces meaningful variations in water protons relaxation. To be more effective, this process should convert a "relaxometrically silent" species into a "relaxometrically active" species.

It has now been found that this objective can be obtained by a method comprising administering insoluble particles containing paramagnetic complexes of Lanthanide or transition metal chelates, as MR-Imaging Probes, in form of particulate which is internalised by cells where they are degraded enzymatically or by effectors in the environment surrounding them, giving rise to water soluble MR-Imaging Probes.

The particles of the invention may bear hydrophobic substituents bound to the surface of the chelating cage so as to make them insoluble.

Said hydrophobic substituents comprise for example aliphatic chains conjugated to the paramagnetic complex through an ester or amide bond.

The particles may alternatively consist of an insoluble macromolecular component (e.g. chitosan or derivatives) to which the paramagnetic complexes are covalently bound or into which the paramagnetic complex is entrapped inside the macromolecular network through non-covalent interactions.

Preferably, the paramagnetic complex is a Gd(III) chelate, a Mn(II) or a Mn(III) chelate.

Non-covalent interactions with the macromolecular component may be obtained by using paramagnetic complexes endowed with a residual negative charge, for instance Gd(III), Mn(II) or Mn(III) chelates endowed with a residual negative charge.

Alternatively, the Gd complexes particle - similarly to the procedures used for particles based on iron oxides - can be partially or completely covered with a polymeric substrate such as dextran or other materials. This treatment on the particulate surface has also the function of controlling the residence time of the system in the blood circuit. The material covering the particulate can be functionalised with one or more synthons able to recognise at molecular level specific units present on the surface of the target cell. After recognition, internalisation occurs analogously to what observed with suitably functionalised iron oxides (for example in the case of binding to transferrin receptor).

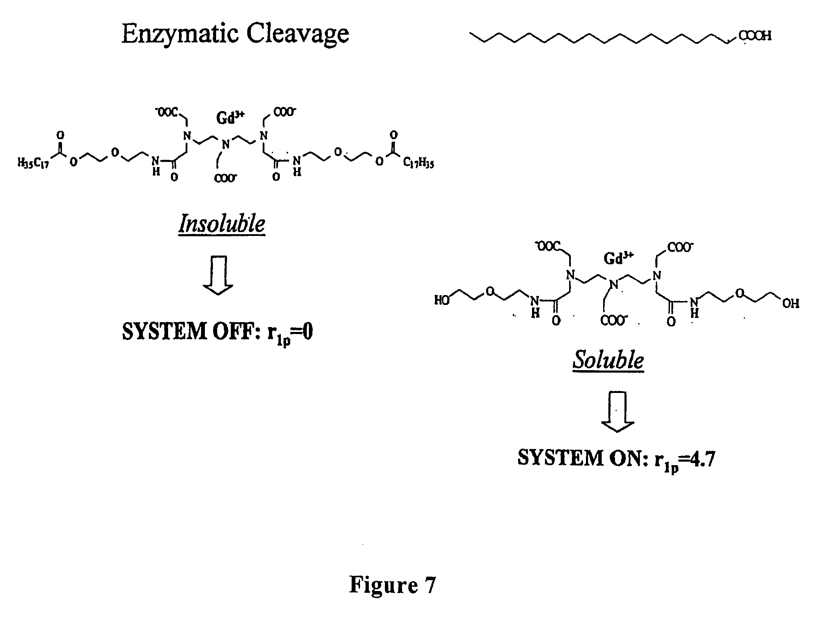

When a particle containing paramagnetic chelates ("relaxometrically silent") is degraded by a suitable enzyme, single units of paramagnetic chelates are released. This process can be easily monitored because the release of paramagnetic chelates determines a progressive reduction of water proton longitudinal relaxation time. This fact is exemplified in Fig. 7 for one of the preferred compounds.

In view of these observations, other particles whose solubilisation is promoted by enzymes not naturally occurring in cells, but specifically expressed by molecular biology techniques, can be prepared. The introduction of particles in cells is a conventional technique, since it is routinely used for delivering genic material in cellular transfection processes. For cells unable of phagocytotic activity one can take advantage of pinocytosis processes which are known to be activated through stimulation of cell surface receptors.

Various approaches for the preparation of particles that may subsequently undergo degradation by specific enzymes can be followed. For example, i) a given system can be rendered insoluble by cross-linking with peptidic chains containing specific sequences for the enzyme of interest or ii) insoluble adducts can be formed between macromolecular systems with opposite electric charge (such as polylysine/polyglutamate) optionally functionalised in such a way that they undergo degradation by a specific enzyme or iii) hydrophobic substituents separated from the complex surface by a spacer susceptible to enzyme action can be introduced. Complex units can be covalently bound to the macromolecules or can be entrapped into the particle's network due to specific interactions with the macromolecules themselves.

In addition to enzymes, other effectors able to destroy the particulate can be considered, such as pH, ions, hormones and metabolites of the microenvironment that surrounds the particle.

The introduction of a particle into a cell appears as the most effective way to entrap a large number of paramagnetic complex units. Once internalized, the particle will be progressively destroyed by enzymes or other agents and can thus form a soluble pool of contrast agent units. This approach can be of very general utility. One example deals with monitoring proliferative processes starting from mother cells wherein the particulate has been internalised. Cellular division processes and delineation of regions wherein resulting cell progenies localise are particularly important in applications concerning stem cells.

Other examples of applications might concern the localisation of cells with macrophagic activity which are highly relevant in the formation of atherosclerotic plaques and in other pathologies.

As an example, a particle based on Gd(III) complexes is represented by a GdDTPA derivative wherein two carboxy groups have been derivatized with hydrophobic chains that make the system insoluble.

The long aliphatic chains are bound to the Gd(III) chelate surface by an ester function, which is hydrolysed by esterase, thus releasing soluble derivatives of the Gd(III) complex and progressively disrupting the particulate as schematized in the enclosed Figure 7. Since the amount of soluble complex (which significantly contributes to increase the relaxation rate of water protons, thus creating a contrast in the resulting MRI image by T1-weighed sequence) depends on the amount of enzyme, the method may provide a contrast agent acting as a reporter of the enzymatic activity in the concerned cell.

As an additional example, an analogous system containing peptidic bonds in place of the ester functionality has been prepared and their cleavage by a proper proteinase enzyme assessed.

### Example 1

### Synthesis of aliphatic chains-disubstituted DTPA and complex thereof with GD(III)

The ligand was synthesised by reaction of DTPA-bisanhydride with H₂N-CH₂-CH₂-O-CH₂-CH₂-OOC-C₁₇H₃₅. The ligand (slightly soluble in water) was complexed with GdCl₃ to give the insoluble chelate that precipitates as white powder (complex Gd-1).

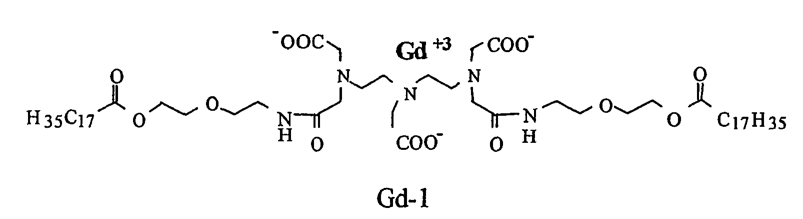

### Example 2

### "In Vitro" evaluation of esterase activity on Complex Gd-1

The activity of two different enzymes (esterase from swine liver [EC3.1.1.1] and lipase from Chromobacterium viscosum [EC 3.1.1.34]) was evaluated on Gd-1 particles. In the presence of the enzyme, in HEPES buffer 50 mM at pH = 8 and 310K, the suspension of Gd-1 shows a progressive decrease in turbidity, which suggests the formation of a soluble Gd(III) complex.

This behaviour was confirmed by measurements of the water protons relaxation rate (R₁) on a NMR spectrometer (Stelar Spinmaster) operating at 0.5T and 298K.

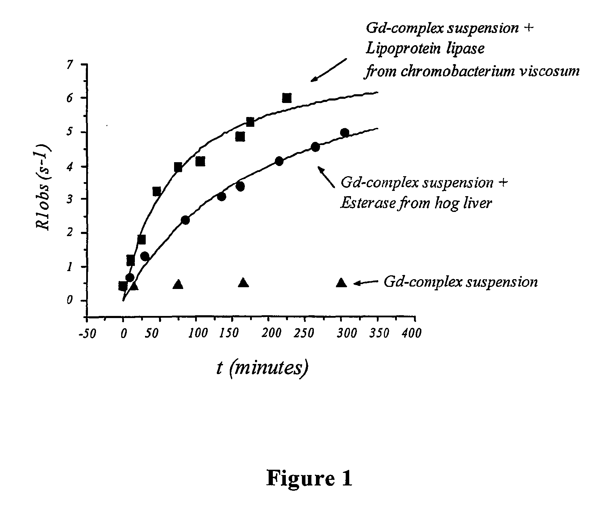

As shown in Figure 1, in the absence of enzyme, R₁ does not change and its value is not different from that of pure water (0.38 s⁻¹), which reflects the limited contribute of the Gd-complex on the particles surface and/or of the small amount of the complex that might be dissolved.

In the presence of the esterase (2 mg) R1 rapidly rises and after 2-3 hours reaches the value expected for a soluble Gd(III) complex resulting from the hydrolysis of the ester bonds of the Gd-1 complex. The differences in the two observed curves, and therefore the different reaction kinetics, can be ascribed to the different amounts of active units per mg of the two enzymes added to the Gd complex suspension.

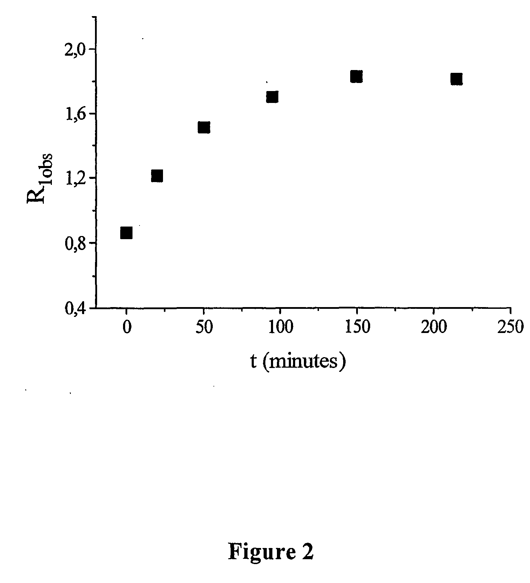

Afterwards, the macrophages esterase was tested. For this purpose, about 4x10⁶ macrophages were subjected to lysis by sonication and about 1 mg Gd-1 was added to the lysate. The enzyme present in the cellular lysate rapidly cleaves the hydrophobic ester groups, thus releasing the soluble Gd complex. The formation of soluble Gd complex is well followed by the increase of water proton relaxation rate (Fig. 2). After about two hours the enzymatic reaction reaches the steady state.

### Example 3

### Phagocytic uptake of Gd-1 on U937 monocytes.

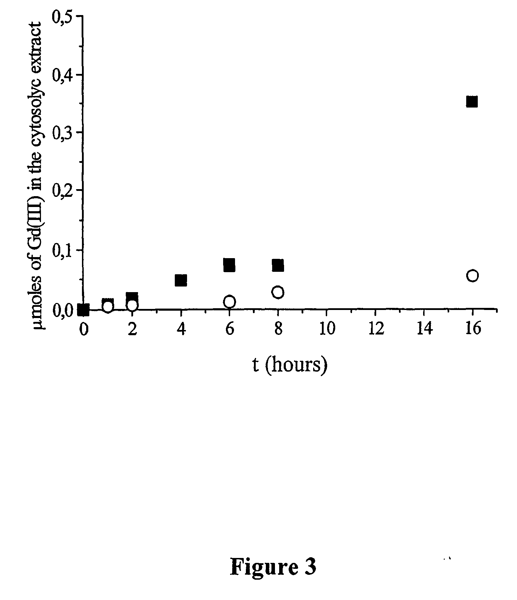

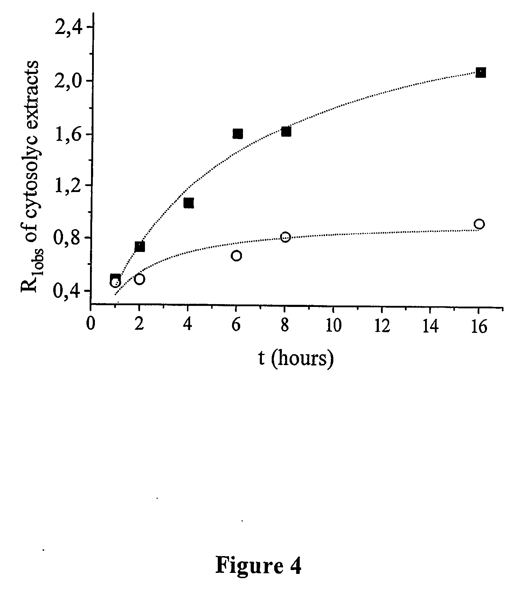

The phagocytic uptake of the Gd-1 particles was carried out on U937 monocytes previously treated for three days with TPA to induce cell differentiation. The size of the Gd-1 particles was determined by electron microscope observation and the measured diameter ranged from 0.5 to 10 µm. Most particles had diameter < 10 µm, which is sufficiently small to allow the phagocytic process. The uptake was carried out at 37°C and the cells were incubated with two different amounts of Gd-1 at subsequent times (1 to 12 hours). After incubation at 37°C for several hours, the medium was removed and the cells were washed three times with 5 mL PBS. In these conditions, Gd-1 particles suspended in the medium are not solubilised; in fact, the relaxivity of the medium remains very close to that of pure water (0.45 s⁻¹). The amount of Gd(III) detected in the cells is directly proportional to incubation time and in 12 hours the saturation of the kinetic curve is not reached (Fig. 3). As expected, the amount of phagocyted Gd(III) is proportional to the amount of complex added to the culture medium at time zero. It is interesting to compare this behaviour with the water proton relaxation rate in the cytosolic extract. This was obtained by freezing three times the cells at -80°C after completion of phagocytosis. The relaxation rate in the cytosolic extract is proportional to the incubation time, but it tends to a plateau (Fig. 4), contrary to what observed in the case of the total amount of phagocyted Gd(III). The large amount of internalised complex probably inhibits the action of the enzymes involved in the hydrolysis.

A control experiment consisting in the uptake of a Gd soluble complex by macrophages was then carried out in order to ascertain whether the Gd internalysed into the cells derives from any Gd-1 dissolved in the medium.

For this purpose different amounts of Gd-HPDO3A (commercially available) were incubated with cells in the same conditions as those used in the presence of Gd-1 particles. The relaxation rate of water protons measured in the cytosolic extract indicates the complete absence of Gd-HPDO3A in the cells. In this way it was demonstrated that the macrophages surface lacks specific or aspecific receptors able to internalise a complex such as Gd-HPDO3A.

### Example 4

### "In vivo" evaluation of intracellular esterase activity on Gd-1

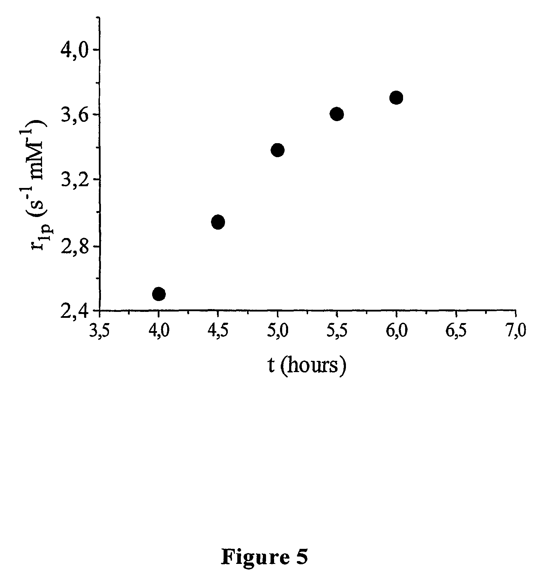

An experiment for evaluating the enzymatic activity inside intact cells was set up, using Gd-1 particles as probes. For this purpose, different capsules containing about 2 x 10⁶ cells (U937 monocytes differentiated to macrophages as described above) were incubated with ca. 5 mg of Gd-1 particles suspended in 5 mL medium (RPMI) for four hours at 37°C. After this period the cells were washed three times with PBS, covered with further 5 ml RPMI and re-incubated for different times at 37°C. In this way it was possible to isolate the contribution deriving from the esterase-catalysed hydrolysis of Gd-1 particles for the time interval that depends on the internalisation step. The proton relaxation rate was measured at successive time intervals (0.5, 1, 2 hours). After 4 hours incubation in the presence of Gd-1 particles the cells internalise about 60 nmol complex, but only 50% of the internalised particles is hydrolysed in this time and contributes to the total relaxivity. In fact, the relaxivity of the cytosolic extract is 2.5 when the cells are resuspended in a medium devoid of Gd-1 and raises to 3.7 in the following two hours of enzymatic hydrolysis. It can be therefore assumed that 80% of Gd-1 internalised particles have been solubilised by cellular esterases (Fig. 5).

### Example 5

### Preparation of small particles by an ultrasound treatment

In order to prepare smaller particles of Gd-1, the gross particles suspension has been undergone to ultrasound treatment. The sonochemical apparatus is a TEKIMP model (Castelfranco Veneto Tv, Italy). An aqueous suspension of Gd-1 (50 mg/ 7 ml) has been prepared in the teflon tube of apparatus. The conditions of sonication were the following: frequency: 18.2 KHz, power: 250W, time: 10min. The suspension is maintained at a temperature < 5°C by a cooling system. Then the sonicated suspension has been filtered (cut off 0.8 µM). The content of Gd in the filtrate corresponds to 10-20% of the total Gd contained in the starting suspension.

### Example 6

### Labelling of Neutrophiles with Gd-1

One of the applications for labelled cells deals with the identification of infection loci in patients affected by fever of unknown origin. This imply the labelling of Neutrophiles obtained from the standard procedures from a specimen of blood drawn from the patient. After labelling the neutrophiles have to be re-injected in the patient who is subjected to MRI examination.

The labelling of neutrophiles (ca. 2.5 x10⁶) has been carried out by incubating them (5 ml) in a medium containing 0.4 mg of particles of Gd-1 prepared according to example 5.

After 2.5 h of incubation at 37°C, the cells were harvested, whashed several times with PBS, then analyzed. The content of Gd per mg protein is 8.8×10⁻⁸ moles, i.e. an amount that after the intracellular degradation of the particle, is well sufficient to allow the visualization of the labelled cells by MRI techniques.

### Example 7

### Labelling of Macrophages with Gd-1 after ultrasound treatment

The particles obtained by the sonication procedure described in example 5 has been tested in an internalization assay with the U937 cells endowed with macrophagic activity. The incubation medium contains 0.2 mg of Gd-1 (total). After ca. 4 hours of incubation the amount of Gd internalized reached a maximum value of 2x10⁻⁸ moles/ mg of protein. This means that ca 10% is taken up by the cells. Such amount is well sufficient to allow the visualisation of these cells. Once the enzymatic degradation process proceeds the signal intensity progressively increases to reach a steady state which lasts for a time long enough for the type of applications of this protocol.

### Example 8

### Synthesis of Gd-2

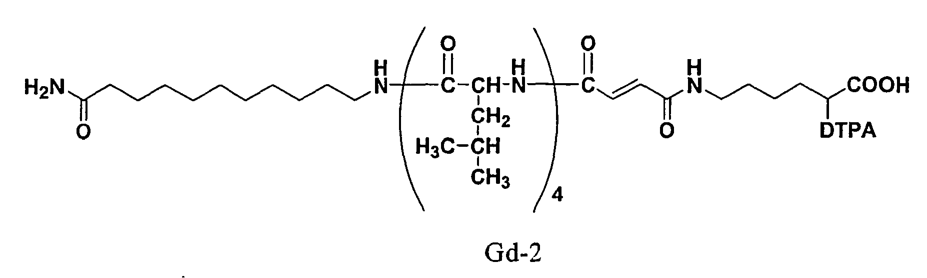

The product was prepared by solid phase synthesis, using Rink Amide resin.

The amino group of 11-aminoundecanoic acid was previously Fmoc protected then bound to the resin with the classical Fmoc-a.a. protocol for Solid Phase Peptide Synthesis.

To the swelled resin in DMA, 6 consecutive couplings (N-Fmoc-11-amminoundecanoic acid, N-Fmocleucinex 4, and maleylamido-LISINO-derivative) were carried out in the presence of PyBop, HOBt and DIPEA.

The Fmoc- deprotection was done with piperidine and the final cleavage of the resin with Reagent B. The row material was washed with DMA, CH₂Cl₂ and diethyl ether and purified by HPLC.

The maleoyl derivative of the LIS-DTPA was obtained by simple reaction with maleyc anhydride in CH₂Cl₂ of the Known LIS-DTPA ligand. The Gd-complex has been obtained by the usual procedure with GdCl3. The G-complex is a pale yellow solid insoluble in water.

### Example 9

### "In vitro" evaluation of proteinase activity on Gd-2 particles

To a suspension of Gd-2 in water (30 mg/ml) a small aliquot (ca 1 mg) of the proteolytic enzyme MMP-12 has been added. Such enzyme is expected to cleave peptidic bonds. The suspension is stirred at ambient temperature for 1h. Then an aliquot (0.1 ml) of the supernatant has undergone to T1-measurement on a Stelar Spinmaster spectrometer operating at 20 MHz. R1 of 0.85 s⁻¹ has been measured. An analogous experiment was carried out upon a prolonged reaction time (4 h) and R1 was found to be 1.8 s-1. This observation can be explained in terms of the cleavage of the insolubilizing synthon with the release of a soluble Gd-chelate.

### Example 10

### Biodegradable Particles made by an insoluble macromolecular system entrapping soluble Gd chelates.

The nanoparticle can be formed by an insoluble matrix which entraps soluble Gd chelates. The matrix is biodegradable by the action of specific enzymes. As the intracellular degradation proceeds, a release of soluble Gd-chelate takes place which, in turn, results in an increase of signal in the resulting MR images.

An example of this class of biodegradable particles for MRI applications is obtained by forming nanoparticles of chitosan and Gd-DTPA. By following well established procedures for the formation of nanoparticles (mean diameter around 250 nm) it has been possible to entrap ca. 6-9 mg of Gd per 100 mg of chitosan.

5 mg of nanoparticles of chitosan entrapping Gd-DTPA has been added to a Petri disk containing 4×10⁶ U937 cells. After 6 h of incubation at 37°C, the cells are harvested and washed several times with PBS. Then the amount of internalized Gd has been determined by ICP. It has been found that the amount of internalized Gd (9.8x 10⁻⁸ moles / mg of protein) is well sufficient for the MRI visualisation.

## Claims

1. In vitro method of cellular labelling, wherein insoluble particles comprising paramagnetic complexes of Lanthanide or transition metal chelates as MR-Imaging Probes are administered in form of particulate, wherein the particulate is internalised by cells and wherein, once internalised, the particles are degraded enzymatically or by effectors in the environment surrounding them, giving rise to water soluble MR-Imaging Probes.

2. The method according to claim 1, wherein the particles are insoluble due to hydrophobic substituents bound to the surface of the chelating cage.

3. The method according to claim 2, wherein the hydrophobic substituents on the particles are aliphatic chains conjugated to the paramagnetic complex through an ester or amide bond.

4. The method according to claim 1, wherein the particles are insoluble due to a macromolecular component forming the particles themselves.

5. The method according to claim 4, wherein the paramagnetic complexes are covalently bound to the macromolecular component.

6. The method according to claims 1-5, wherein the paramagnetic complex is a Gd(III) chelate.

7. The method according to claims 1-5, wherein the paramagnetic complex is a Mn(II) or a Mn(III) chelate.

8. The method according to claim 4, wherein the insoluble macromolecular component is chitosan or derivatives thereof.

9. The method according to claim 8, wherein the paramagnetic complex is entrapped inside the macromolecular network through non-covalent interactions.

10. The method according to claim 9, wherein the paramagnetic complex is a Gd(IXX) chelate endowed with a residual negative charge.

11. The method according to claim 9, wherein the paramagnetic complex is a Mn(II) or a Mn(III) chelate endowed with a residual negative charge.

12. The method according to claims 1-11, wherein the particles are covered by a dextran polymer or other suitable material to favour the formation of stable suspensions and to increase the lifetime of the particles in blood.

13. The method according to claims 1-11, wherein the particles are functionalised with synthons able to target them to interact with specific recognition sites on the outer membrane of the cells of interest, thus stimulating their cell-internalization.

14. The method according to any one of claims 1-13, wherein the insoluble particles comprise a compound of formula: 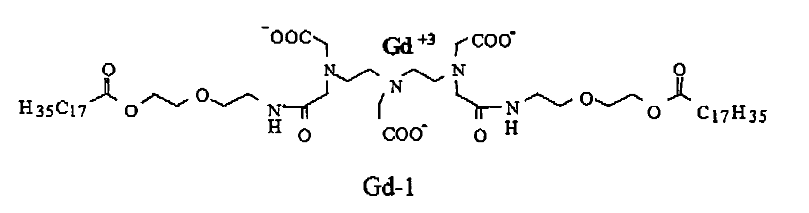 or a compound of formula:

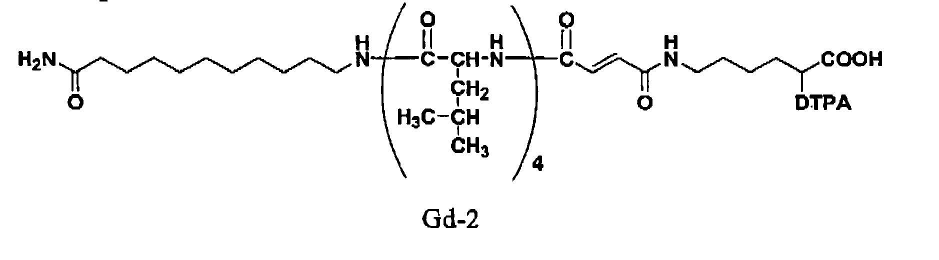

## Patentansprüche

1. In-vitro-Verfahren zur Zellmarkierung, wobei unlösliche Partikel, umfassend paramagnetische Komplexe von Lanthanid- oder Übergangsmetallchelaten, als MR-Bildgebungssonden in Form von Partikulat verabreicht werden, wobei das Partikulat durch Zellen internalisiert wird und wobei, sobald es internalisiert ist, die Partikel enzymatisch oder durch Effektoren in der sie umgebenden Umgebung abgebaut werden, wodurch wasserlösliche MR-Bildgebungssonden entstehen.

2. Verfahren nach Anspruch 1, wobei die Partikel aufgrund hydrophober Substituenten, gebunden an die Oberfläche des chelatbildenden Käfigs, unlöslich sind.

3. Verfahren nach Anspruch 2, wobei die hydrophoben Substituenten auf den Partikeln aliphatische Ketten sind, mit dem paramagnetischen Komplex durch eine Ester- oder Amidbindung konjugiert.

4. Verfahren nach Anspruch 1, wobei die Partikel aufgrund einer makromolekularen Komponente, die die Partikel selbst bildet, unlöslich sind.

5. Verfahren nach Anspruch 4, wobei die paramagnetischen Komplexe an die makromolekulare Komponente kovalent gebunden sind.

6. Verfahren nach den Ansprüchen 1-5, wobei der paramagnetische Komplex ein Gd(III)-Chelat ist.

7. Verfahren nach den Ansprüchen 1-5, wobei der paramagnetische Komplex ein Mn(II)- oder ein Mn(III)-Chelat ist.

8. Verfahren nach Anspruch 4, wobei die unlösliche makromolekulare Komponente Chitosan oder Derivate davon ist.

9. Verfahren nach Anspruch 8, wobei der paramagnetische Komplex im Inneren des makromolekularen Netzwerks durch nichtkovalente Wechselwirkungen eingeschlossen ist.

10. Verfahren nach Anspruch 9, wobei der paramagnetische Komplex ein Gd(III)-Chelat, ausgestattet mit einer negativen Restladung, ist.

11. Verfahren nach Anspruch 9, wobei der paramagnetische Komplex ein Mn(II)- oder ein Mn(III)-Chelat, ausgestattet mit einer negativen Restladung, ist.

12. Verfahren nach den Ansprüchen 1-11, wobei die Partikel mit einem Dextranpolymer oder anderen geeigneten Material bedeckt sind, um die Bildung von stabilen Suspensionen zu begünstigen und die Lebensdauer der Partikel im Blut zu erhöhen.

13. Verfahren nach den Ansprüchen 1-11, wobei die Partikel mit Synthonen funktionalisiert sind, fähig, sie zum Wechselwirken mit spezifischen Erkennungsstellen auf der äußeren Membran der Zellen von Interesse zu targetieren, wodurch deren Zell-Internalisierung stimuliert wird.

14. Verfahren nach einem der Ansprüche 1-13, wobei die Partikel eine Verbindung der Formel 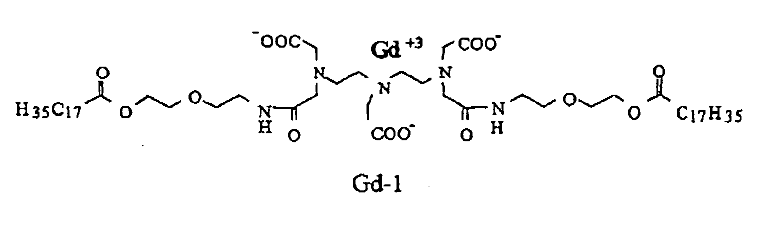 oder eine Verbindung der Formel 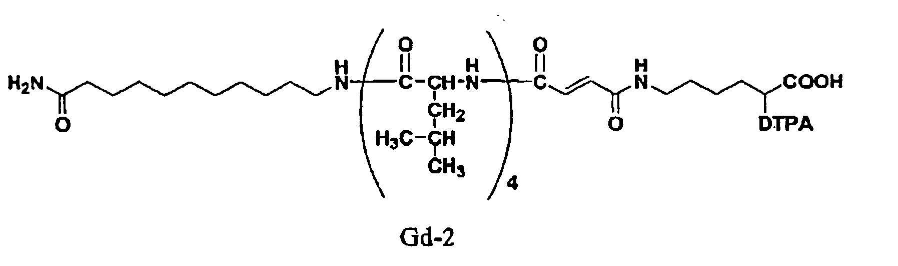 umfassen.

## Revendications

1. Procédé in vitro de marquage cellulaire, dans lequel des particules insolubles comprenant des complexes paramagnétiques de Lanthanide ou de chélates de métaux de transition comme sondes d'imagerie-RM sont administrées sous forme de particule, dans lequel la particule est internalisée par les cellules et dans lequel, une fois internalisées, les particules sont dégradées enzymatiquement ou par des effecteurs dans l'environnement les entourant, donnant naissance à des sondes d'imagerie-RM hydrosolubles.

2. Procédé selon la revendication 1, dans lequel les particules sont insolubles en raison de substituants hydrophobes liés à la surface de la cage de chélation.

3. Procédé selon la revendication 2, dans lequel les substituants hydrophobes sur les particules sont des chaînes aliphatiques conjuguées au complexe paramagnétique par une liaison ester ou amide.

4. Procédé selon la revendication 1, dans lequel les particules sont insolubles en raison d'un composant macromoléculaire formant les particules elles-mêmes.

5. Procédé selon la revendication 4, dans lequel les complexes paramagnétiques sont liés par liaison covalente au composant macromoléculaire.

6. Procédé selon les revendications 1 à 5, dans lequel le complexe paramagnétique est un chélate de Gd(III).

7. Procédé selon les revendications 1 à 5, dans lequel le complexe paramagnétique est un chélate de Mn(II) ou un chélate de Mn(III).

8. Procédé selon la revendication 4, dans lequel le composant macromoléculaire insoluble est le chitosane ou des dérivés de celui-ci.

9. Procédé selon la revendication 8, dans lequel le complexe paramagnétique est piégé à l'intérieur du réseau macromoléculaire par des interactions non covalentes.

10. Procédé selon la revendication 9, dans lequel le complexe paramagnétique est un chélate de Gd(III) doté d'une charge négative résiduelle.

11. Procédé selon la revendication 9, dans lequel le complexe paramagnétique est un chélate de Mn(II) ou un chélate de Mn(III) doté d'une charge négative résiduelle.

12. Procédé selon les revendications 1 à 11, dans lequel les particules sont recouvertes par un polymère de dextrane ou un autre matériau approprié afin de favoriser la formation de suspensions stables et d'augmenter la durée de vie des particules dans le sang.

13. Procédé selon les revendications 1 à 11, dans lequel les particules sont fonctionnalisées avec des synthons capables de les cibler pour interagir avec des sites de reconnaissance spécifiques de la membrane externe des cellules d'intérêt, stimulant ainsi leur internalisation cellulaire.

14. Procédé selon l'une quelconque des revendications 1 à 13, dans lequel les particules insolubles comprennent un composé de formule : 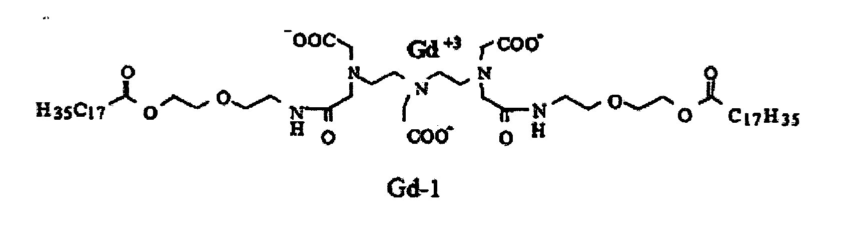 ou un composé de formule :
